(19) Europäisches Patentamt
European Patent Office
Office européen des brevets

(11) Publication number : **0 556 585 A2**

(12) # EUROPEAN PATENT APPLICATION

(21) Application number : **93100946.8**

(22) Date of filing : **22.01.93**

(51) Int. Cl.[5] : **C07D 491/22,** A61K 31/47, // (C07D491/22, 317:00, 311:00, 225:00, 221:00, 209:00)

(30) Priority : **24.01.92 JP 11424/92**
**10.12.92 JP 330296/92**

(43) Date of publication of application :
**25.08.93 Bulletin 93/34**

(84) Designated Contracting States :
**AT BE CH DE DK ES FR GB GR IE IT LI LU NL PT SE**

(71) Applicant : **TAKEDA CHEMICAL INDUSTRIES, LTD.**
**1-1, Doshomachi 4-chome**
**Chuo-ku, Osaka 541 (JP)**

(72) Inventor : **Akimoto, Hiroshi**
**4-25, Morikitamachi 6-chome**
**Higashinada-ku, Kobe, Hyogo 658 (JP)**
Inventor : **Ootsu, Koichiro**
**9-26, Todaiji 3-chome**
**Shimamoto-cho, Mishima-gun, Osaka 618 (JP)**
Inventor : **Kawamura, Noriaki**
**1-3-307, Doshodai 1-chome**
**Suma-ku, Kobe, Hyogo 654-01 (JP)**

(74) Representative : **von Kreisler, Alek et al**
**Patentanwälte von Kreisler-Selting-Werner,**
**Postfach 10 22 41, Bahnhofsvorplatz 1**
**D-50462 Köln (DE)**

(54) **Condensed camptothecin derivatives their production and use as antitumor agents.**

(57) Novel compound represented by the formula :

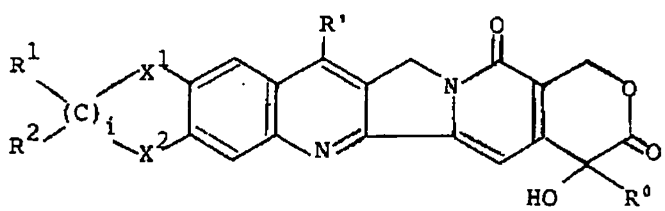

(I)

wherein R' stands for an aliphatic hydrocarbon group substituted with a hydrophilic group ; $R^0$ stands for a lower alkyl group ; $R^1$ and $R^2$ independently stand for a hydrogen atom or an optionally substituted lower hydrocarbon group ; $X^1$ and $X^2$ independently stand for -O-, $-S(O)_K-$ (K denotes a whole number of 0 to 2),

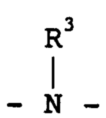

($R^3$ stands for a hydrogen atom or an optionally substituted lower hydrocarbon group) or an optionally substituted methylene group : i denotes a whole number of 1 to 3 ; and $R^1$ and $R^2$ may be different from each other at the repetition of the number i, or a salt thereof which has a topoisomerase I inhibition activity and is useful as an antitumor agents.

EP 0 556 585 A2

This invention relates to novel condensed heterocyclic derivatives which are useful as an antitumor agent, their production and use.

Camptothecin and its analogues extracted and isolated from, for example, Camptotheca acuminata Nyssaceae, are compounds contained in the category of pentacycloalkaloids. The principal action mechanism of those compounds is, unlike known antitumor agents, to inhibit topoisomerase I which plays an important role in the synthesis of nucleic acid. There is a report that those compounds do not show cross-tolerance with conventional drugs but display unique and potent antitumor effects. Camptothecin itself was subjected to Phase II trials in the Untied States, but the development as medicines was discontinued, because severe toxicities were observed and the efficacy was not so desirable as expected. Further, camptothecin is very hardly soluble in water, which inevitably causes great restriction in its formulation into pharmaceuticals and using clinically. [F. M. Muggia et al., Cancer Chemother. Rep., 56, 515 (1972) and C. R. Hutchinson, Tetrahedron, 37, 1047 (1981)].

With the purpose of solving these problems, studies have been conducted on derivatives employing pentacyclo-condensed ring of camptothecin [JP-91068007 and J. Med. Chem., 34, 98 (1991)] and derivatives expanded to hexacyclo-condensed ring compounds [JP-90138186 and M. E. Wall et al., EP-418099-A].

What is now especially desired in the field of cancer therapy is the creation of a drug showing a highly selective toxicity against tumor cells by a new action mechanism, being conveniently handled in the clinical field and having excellent therapeutic effects. Camptothecin derivatives so far studied have strong toxicity on the digestive system and marrow and are relatively poor in water-solubility, thus they are hardly satisfactory from the viewpoint of practical use.

Taking the above-mentioned circumstances into consideration, the present inventors have conducted diligent studies. As a result, they found that the derivatives prepared by introducing a water-soluble substituent into a specific position of a condensed heterocyclic compound inhibit, unexpectedly, specifically one or two enzymes playing a role in topoisomerism in the system of nucleic acid synthesis, display a highly selective toxicity against various tumor cells (especially, e.g. cells of the lung cancer of man), overcome the problem of relatively poor water-solubility , and show excellent therapeutic effects, thus accomplishing the present invention.

The present invention relates to

(1) a compound represented by the general formula:

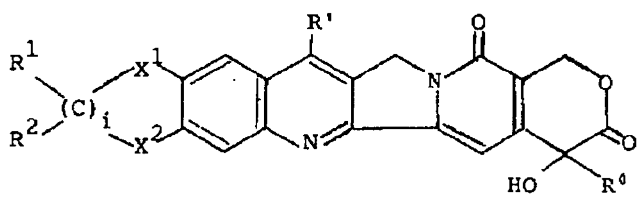

(I)

wherein R' stands for an aliphatic hydrocarbon group substituted with a hydrophilic group; $R^0$ stands for a lower alkyl group; $R^1$ and $R^2$ independently stand for a hydrogen atom or an optionally substituted lower hydrocarbon group; $X^1$ and $X^2$ independently stand for -O-, $-S(O)_k-$ (k denotes a whole number of 0 to 2),

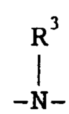

($R^3$ stands for a hydrogen atom or an optionally substituted lower hydrocarbon group) or an optionally substituted methylene group; i denotes a whole number of 1 to 3; and $R^1$ and $R^2$ may be different from each other at the repetition of the number i, or a salt thereof,

(2) a compound as described above in (1), which is a compound of the general formula:

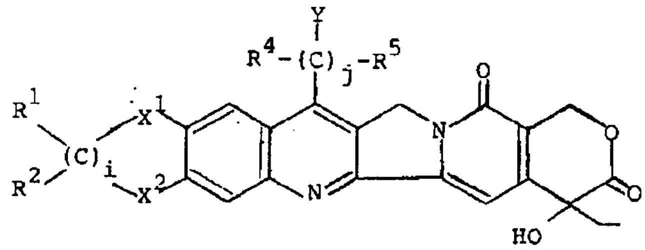

(II)

wherein $R^1$, $R^2$, $R^4$ and $R^5$ independently stand for a hydrogen atom or an optionally substituted lower hydrocarbon group; $X^1$ and $X^2$ independently stand for -O-, $-S(O)_k-$ (k denotes a whole number of 0 to 2),

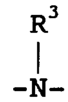

($R^3$ stands for a hydrogen atom or an optionally substituted hydrocarbon group) or an optionally substituted methylene group; Y stands for $-O-R^6$ ($R^6$ stands for a hydrogen atom or an organic group having a water-soluble substituent) or

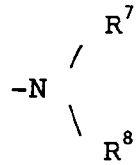

($R^7$ and $R^8$ independently stand for a hydrogen atom, an optionally substituted lower hydrocarbon group or an organic group having a water-soluble substituent, or they may form a heterocyclic group taken together with the adjacent nitrogen atom; i and j respectively denote a whole number of 1 to 3); $R^1$, $R^2$, $R^4$ and $R^5$ may be different from one another at the repetition of the number i or j, or a salt thereof.

(3) a method of producing a compound as described above in (1), which comprises reacting a compound represented by the formula:

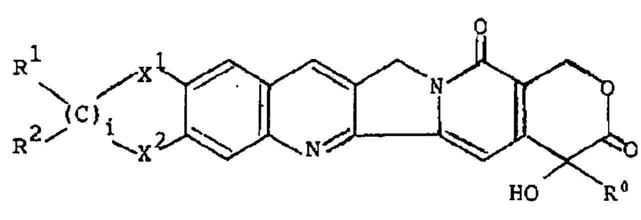

(III)

wherein $R^0$, $R^1$, $R^2$, $X^1$, $X^2$ and i are of the same meaning as defined above in (1), or a salt thereof with a compound represented by the formula:

R' - Z (IV)

wherein R' is the same meaning as defined above in (1); Z stands for a hydrogen atom, $CH_2OH$, CHO or COOH, or a salt thereof or a reactive derivative thereof,

(4) a method of producing a compound as described above in (1), which introduces hydroxyl group into a compound of the general formula:

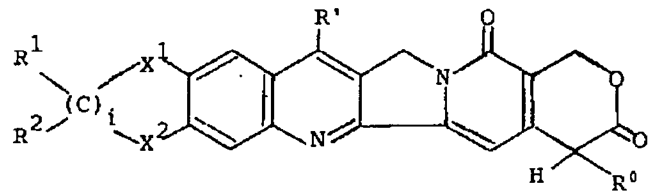

(V)

wherein symbols are of the same meaning as defined above in (1), or a salt thereof,

(5) an antitumor composition containing a compound as described above in (1).

In the compounds of the present invention, there exist, because of the formation of asymmetric carbon due to the substituent on the lactone ring, compounds having the absolute configurations of S and R. Among them, the racemic compounds (a mixture of compounds of R and S absolute configurations) and optically active compounds having the absolute configuration S are preferable. The asymmetric center can be in any other position depending on the kinds of substituents, and the absolute configuration may be any of S, R or a mixture of R and S. When there exists an optically active compound or a diastereoisomer, it can be isolated by a conventional separation and purification method.

In the above formulas, R' stands for an aliphatic hydrocarbon group substituted with a hydrophilic group. Examples of the hydrophilic group include the follow: -OQ, -CN, $-NQ_2$, $-\overset{\oplus}{N}Q_3$, an cyclic amino, -OCOQ, -OCONHQ, -COOQ, -NHCOQ, $-NHSO_2Q$, $-NHPO_2Q$, -NHCONHQ, -NHCOOQ, $-SO_3Q$, $-OSO_3H$, etc., wherein Q stands for a hydrogen atom or an optionally substituted lower hydrocarbon group: The preferable examples of R' include the follow: -OQ, $-NQ_2$, $-\overset{\oplus}{N}Q_3$, an cyclic amino (eg. 5- to 8-membered cyclic amino group which may include 1 to 3 hetero atoms of oxygen atom or sulfur atom other than nitrogen atom; such as pyrrolidino, pyperidino, morpholino, etc.), -OCOQ, -OCONHQ, -COOQ, -NHCOQ, - NHCONHQ and -NHCOOQ wherein Q is as defined above. The more preferable examples of R' include -OQ, $-NQ_2$, - NHCOQ and -NHCOOQ wherein Q is as defined above.

Examples of the lower hydrocarbon group for Q include a $C_{1-6}$ alkyl (e.g. methyl, ethyl, propyl, isopropyl, butyl, tert-butyl), $C_{2-6}$ alkenyl (e.g. vinyl, 1-methylvinyl, 1-propenyl, aryl, allenyl), $C_{2-6}$ alkynyl (e.g. ethynyl, 1-propynyl, propargyl), $C_{3-6}$ cycloalkyl (e.g. cyclopropyl, cyclopentyl, cyclohexyl), phenyl or benzyl, etc., preferably a $C_{1-4}$ alkyl group (e.g. methyl) or a benzyl. These lower hydrocarbon groups of Q may have one to three substituents at any position where substitution can take place. Examples of these substituents include a hydroxy, carboxy, amino, mono-$C_{1-6}$ alkyl amino (e.g. methylamino, ethylamino, propylamino, diethylamino, dipropylamino, diisopropylamino, dibutylamino), 5- to 6- cyclicamino (e.g. pyrolyl, piperidino, morpholino, pyridyl, N-methylpiperazinyl, N-ethylpiperazinyl), $C_{1-3}$ alkoxy-$C_{1-3}$ alkyl (e.g. methoxymethyl, ethoxymethyl, 2-methoxyethyl, 2-ethoxyethyl), sulfo, sulfino, phosphono, dihydroxyboryl, etc.

Examples of the aliphatic hydrocarbon group or R' include a straight-chain or branched $C_{1-11}$ alkyl group, preferably $C_{1-6}$ alkyl group (e.g. methyl, ethyl, n-propyl, iso-propyl, n-butyl, iso-butyl, tert-butyl, n-pentyl, n-hexyl etc.), a straight-chain or branched $C_{2-4}$ alkenyl group (e.g. vinyl, allyl, 2-butenyl, 2,4-butadienyl, etc.) or a straight-chain or branched $C_{2-4}$ alkynyl group (e.g. propynyl, propargyl, 2-butynyl, etc.). Preferable example of the aliphatic hydrocarbon group for R' is a straight-chain or branched $C_{1-6}$ alkyl group.

More practically, R', which stands for an aliphatic hydrocarbon group substituted with a hydrophilic group, includes a group of the formula:

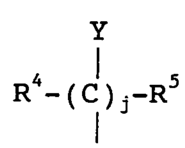

wherein $R^4$ and $R^5$ independently stand for a hydrogen atom or an optionally substituted lower hydrocarbon group; Y stands for $-O-R^6$ ($R^6$ stands for a hydrogen atom or an organic group having a water-soluble substituent) or

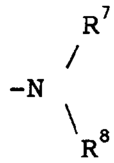

($R^7$ and $R^8$ independently stand for a hydrogen atom, an optionally substituted lower hydrocarbon group or an organic group having a water-soluble substituent, or they may form a heterocyclic group taken together with the adjacent nitrogen atom); and j denotes a whole number of 1 to 3.

$R^0$ stands for a lower alkyl group. Examples of the lower alkyl group shown by $R^0$ include a $C_{1-6}$ alkyl group (e.g. methyl, ethyl, propyl, iso-propyl, butyl, tert-butyl). Among them, a $C_{1-3}$ alkyl group (e.g. methyl, ethyl, propyl, iso-propyl) are preferable, especially ethyl group is most preferable.

$R^1$, $R^2$, $R^4$ and $R^5$ stand for a hydrogen atom or an optionally substituted lower hydrocarbon group. Examples of the lower hydrocarbon groups shown by $R^1$, $R^2$, $R^4$ and $R^5$ include the same lower hydrocarbon group as defined in Q. The lower hydrocarbon groups of $R^1$, $R^2$, $R^4$ and $R^5$ may have one to three substituents at any position where substitution can take place. Examples of these substituents include a halogen (e.g. chlorine, bromine, fluorine, iodine), nitro, cyano, hydroxy, carboxy, carbamoyl, N-mono-$C_{1-6}$ alkylcarbamoyl (e.g. N-methylcarbamoyl, N-ethylcarbamoyl, N-propylcarbamoyl), N,N-di-$C_{1-6}$ alkylcarbamoyl (e.g. N,N-dimethylcarbamoyl, N,N-diethylcarbamoyl, N,N-dipropylcarbamoyl), $C_{1-6}$ alkoxy (e.g. methoxy, ethoxy, propoxy, butyloxy), phenoxy, benzyloxy, amino, mono-$C_{1-6}$ alkyl amino (e.g. methylamino, ethylamino, propylamino), di-$C_{1-6}$ alkyl amino (e.g. dimethylamino, diethylamino, dipropylamino), trifluoromethyl, 5- to 8-membered cyclic amino which may include 1 to 3 hetero atoms of oxygen atom or sulfur atom other than nitrogen atom. (e.g. pyrrolidinyl, pyrrolinyl, morpholino, etc.) $C_{1-5}$ alkanoyl (e.g. formyl, acetyl, propionyl), $C_{1-3}$ alkoxy-$C_{1-3}$ alkyl (e.g. methoxymethyl, ethoxymethyl, 2-methoxyethyl, 2-ethoxyethyl), $C_{1-6}$ alkylthio (e.g. methylthio, ethylthio), sulfo, sulfamoyl, N-mono-$C_{1-6}$ alkylsulfamoyl (e.g. N-methylsulfamoyl, N-ethylsulfamoyl, N-propylsulfamoyl), N,N-di-$C_{1-6}$ alkylsulfamoyl (e.g. N,N-dimethylsulfamoyl, N,N-diethylsulfamoyl, N,N-dipropylsulfamoyl), sulfino, $C_{1-6}$ alkylsulfinyl (e.g. methylsulfinyl, ethylsulfinyl), $C_{1-6}$ alkylsulfonyl (e.g. methylsulfonyl, ethylsulfonyl), phosphono, dihydroxyboryl among others, and these substituents may further have one or two of the above-mentioned substituents on any position where such substitution can take place. The preferably example of these substituents includes hydroxy, carboxy, amino, mono-$C_{1-6}$ alkylamino, di-$C_{1-6}$alkylamino, 5- to 8-membered cyclic amino, $C_{1-3}$ alkoxy-$C_{1-3}$ alkyl, sulfo, sulfino, phosphono and dihydroxyboryl. The preferable example of each of $R^1$, $R^2$, $R^4$ and $R^5$ is a hydrogen atom or a $C_{1-3}$ alkyl group. The most preferable example thereof is a hydrogen atom.

$X^1$ and $X^2$ stand for the formula: -O-, -S(O)$_k$- (k denotes a whole number of 0, 1 or 2),

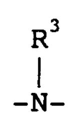

($R^3$ stands for a hydrogen atom or an optionally substituted lower hydrocarbon group) or an optionally substituted methylene group. Examples of the lower hydrocarbon group shown by $R^3$ include lower hydrocarbon groups specifically described in respects of Q, preferably $C_{1-3}$ alkyl groups. And, the substituents on the lower hydrocarbon group for $R^3$ include the number and kinds of substituents specifically described in respects of the lower hydrocarbon group for $R^1$, $R^2$, $R^4$ and $R^5$. And, as the substituents of the optionally substituted methylene groups shown by $X^1$ and $X^2$, the number and kinds of substituents as described in respects of the lower hydrocarbon group for $R^1$, $R^2$, $R^4$ and $R^5$ are likewise mentioned. As $R^3$, a hydrogen atom, a $C_{1-3}$ alkyl group, etc. are preferable and a hydrogen atom, etc. are more preferable. The preferable examples of $X^1$ and $X^2$ are -O-, -S- or -NH-, and more preferable examples thereof are -O-.

In the above formulas, Y stands for -O-$R^6$ ($R^6$ stands for a hydrogen atom or an organic group having a water-soluble substituent) or

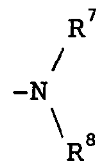

($R^7$ and $R^8$ independently stand for a hydrogen atom, an optionally substituted lower hydrocarbon group or an organic group having a water-soluble substituent, or they may form a heterocyclic group taken together with the adjacent nitrogen atom).

And, examples of the organic group having a water-soluble substituent shown by $R^6$ include such lower hydrocarbon groups having a water-soluble substituent(s) as forming ether, ester or carbamic acid ester taken together with the adjacent oxygen atom, i.e. -O-$R^6$ includes -O$R^{6'}$,

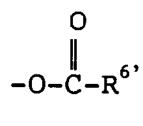

and -OCONH-$R^{6'}$ wherein $R^{6'}$ is a lower hydrocarbon group having a water-soluble substituent(s). The lower hydrocarbon groups for $R^{6'}$ are $C_{1-6}$ alkyl (e.g. methyl, ethyl, propyl, iso-propyl, butyl, tert-butyl), $C_{2-6}$ alkenyl (e.g. vinyl, 1-methylvinyl, 1-propenyl, aryl, allenyl), $C_{2-6}$ alkynyl (e.g. ethynyl, 1-propynyl, propargyl), $C_{3-6}$ cycloalkyl (e.g. cyclopropyl, cyclopentyl, cyclohexyl), phenyl or benzyl, etc., preferably a hydrogen atom, an $C_{1-4}$ alkyl (e.g. methyl, ethyl propyl, iso-propyl) group or an benzyl group. The lower hydrocarbon group for $R^{6'}$ is having, at appropriate positions, 1 to 3 water-soluble substituents. The water soluble substituent on the lower hydrocarbon group for $R^{6'}$ is a hydroxy, carboxyl, amino, mono-$C_{1-6}$ alkylamino (e.g. methylamino, ethylamino, propylamino, isopropylamino, butylamino), di-$C_{1-6}$ alkylamino (e.g. dimethylamino, diethylamino, dipropylamino, diisopropylamino, dibutylamino), 5- to 6-membered cyclic amino (e.g. pyrrolyl, piperidino, morpholino, pyridyl, N-methylpiperazinyl, N-ethylpiperazinyl), $C_{1-3}$ alkoxy-$C_{1-3}$ alkyl (e.g.methoxymethyl, ethoxymethyl, 2-methoxyethyl, 2-ethoxyethyl), sulfo, sulfino, phosphono or dihydroxyboryl, etc., preferably a hydroxy, carboxyl, amino, sulfo, etc.

The preferable examples of -O-$R^6$ are -O$R^{6''}$, -OCO$R^{6''}$ or -OCONH$R^{6''}$ ($R^{6''}$ stands for (i) a hydrogen atom or (ii) a $C_{1-6}$ alkyl, $C_{2-6}$ alkenyl, $C_{2-6}$ alkynyl, $C_{3-6}$ cycloalkyl or benzyl group, these $C_{1-6}$ alkyl, $C_{2-6}$ alkanyl, $C_{2-6}$ alkynyl, $C_{3-6}$ cycloalkyl and benzyl group having one to three water-soluble substituents selected from hydroxy, carboxy, amino, mono-$C_{1-6}$ alkylamino, di-$C_{1-6}$ alkylamino, 5- to 6-membered cyclic amino, $C_{1-3}$ alkoxy-$C_{1-3}$ alkyl, sulfo, sulfino, phosphono, dihydroxyborl, etc.). More preferable examples of -O-$R^6$ includes -O$R^{6'''}$ ($R^{6'''}$ stands for (i) a hydrogen atom or (ii) a $C_{1-4}$ alkyl or benzyl group having one to three substituents selected from hydroxy, carboxy, amino, mono-$C_{1-6}$ alkylamino, di-$C_{1-6}$ alkylamino, 5- to 6-membered cyclic amino, $C_{1-3}$ alkoxy-$C_{1-3}$ alkyl, sulfo, sulfino, phosphono, dihydroxyborl, etc.). Most preferable example of -O-$R^6$ is a hydroxyl group.

Examples of the lower hydrocarbon groups of the shown by $R^7$ and $R^8$ include those specifically described in respects of the lower hydrocarbon group for Q, preferably a $C_{1-4}$ alkyl or benzyl group. And, as the substituents on the lower hydrocarbon group for $R^7$ and $R^8$, while the number and kinds of the substituents described in detail in respects of the lower hydrocarbon group for $R^1$, $R^2$, $R^4$ and $R^5$ are likewise mentioned, preferably a $C_{1-4}$ alkyl group and a benzyl group. And, as the organic group having a water-soluble substituent shown by $R^7$ and $R^8$, mention is made of such an organic group having a water-soluble substituent described in detail in respect of $R^6$ as forming amido, sulfamoyl, phosphoramido, carbamoyl oxycarbonylamino or ureido linkage, taken together with the adjacent nitrogen atom,

i.e, $R^7$ and $R^8$ include

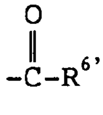, 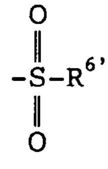, 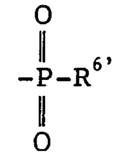, 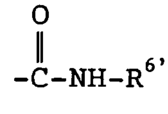, 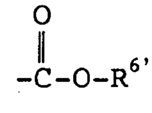

wherein $R^{6'}$ is as defined above.

And, as the heteroxyclic ring formed together with the adjacent nitrogen atom, which is shown by $R^7$ and $R^8$, 5- to 8-membered rings are preferable, as exemplified by pyrrolidinyl, pyrrolinyl, pyrrolyl, imidazolyl, pyrazolyl, imidazolyl, piperidino, morpholino, dihydropyridyl, tetrahydropyridyl, N-methyl piperazinyl, N-ethyl piperazinyl, azacycloheptyl and azacyclooctyl, preferably pyrrolidinyl and morpholins. These heterocyclic rings may have 1 to 3 of the above-mentioned water-soluble substituents for $R^6$ at relevant positions. The preferable examples of $-NR^7R^8$ include - $NR^{7'}R^{8'}$, $-NHCOR^{7'}$, $-NHSO_2R^{7'}$, $-NHPOR^{7'}$, $-NHCONHR^{7'}$, $-NHCOOR^{7'}$ ($R^{7'}$ and $R^{8'}$ independently stand for (i) a hydrogen atom or (ii) a $C_{1-6}$ alkyl (e.g. methyl, ethyl, propyl, isopropyl, butyl, tert-butyl), $C_{2-6}$ alkenyl (e.g. vinyl, 1-methylvinyl, 1-propenyl, aryl, allenyl), $C_{2-6}$ alkynyl (e.g. ethynyl, 1-propynyl, propargyl), $C_{3-6}$ cycloalkyl (e.g. cyclopropyl, cyclopentyl, cyclohexyl), phenyl or benzyl, etc., these $C_{1-6}$ alkyl, $C_{2-6}$ alkenyl, $C_{2-6}$ alkynyl, $C_{3-6}$ cycloalkyl, phenyl and benzyl group having one to three water-soluble substituents selected from the group consisting of hydroxy, carboxy, amino, mono-$C_{1-6}$ alkyl amino (e.g. methylamino, ethylamino, propylamino, diethylamino, dipropylamino, diisopropylamino, dibutylamino), 5- to 6-cyclic amino (e.g. pyrolyl, piperidino, morpholino, pyridyl, N-methylpiperazinyl, N-ethylpiperazinyl), $C_{1-3}$ alkoxy-$C_{1-3}$ alkyl (e.g. methoxymethyl, ethoxymethyl, 2-methoxyethyl, 2-ethoxyethyl), sulfo, sulfino, phosphono, dihydroxyboryl, etc. The more preferable examples of $-NR^7R^8$ are an amino group or $-NHCOR^{7''}$ or $-NHCOOR^{7''}$ ($R^{7''}$, stands for (i) a hydrogen atom or (ii) a $C_{1-4}$ alkyl or benzyl group having one to three water-soluble substituents selected from the group consisting of hydroxy, carboxy, amino, mono-$C_{1-6}$ alkylamino, di-$C_{1-6}$ alkylamino, 5- to 6-membered cyclic amino, $C_{1-3}$ alkoxy-$C_{1-3}$ alkyl, sulfo, sulfino, phosphono and dihydroxyborl.) Most preferable example of $-NR^7R^8$ includes an amino group.

In short, as Y, an amino or hydroxyl group which may optionally be protected with a protecting group are preferable. Examples of the protective group include those described in "Protective Group in Organic Synthesis [T.W.Greene(A Wiley-interscience)], more specifically, formyl group, acetyl group, chloroacetyl group, trifluoroacetyl group, benzoyl group, phthaloyl group, 2,2,2-trichloroethyloxycarbonyl group, tert-butyloxycarbonyl group, benzyloxycarbonyl group or 7-(fluorenyloxycarbonyl group, preferably, tert-butyloxycarbonyl group, benzyloxycarbonyl group or trifluoroacetyl group.

The symbols i and j denote a whole number of 1 to 3, respectively. The preferable example of i is 1, and j is 1 or 2.

The following is the method of producing the compound (I) and (II) or salts thereof of the present invention. The compound (I) and (II) or salts thereof of the present invention can be produced by subjecting the compound (III) or a salt thereof to radical reaction with the compound (IV) or a salt thereof or a reactive derivative thereof. The radical reaction can be conducted, when Z of the compound (IV) is a hydrogen atom, by adding a peroxide thereto in the presence of a strong acid (e.g. conc. sulfuric acid or trifluoroacetic acid) and by causing generation of radical by heating or photo reaction to thereby convert into the radical of the compound (IV). The amount of the compound (IV) to be used ranges generally from about 1 to 200 mol. relative to the compound (III), preferably from about 5 to 50 mol.. As the peroxide, mention is made of, for example, benzoyl peroxide or m-chlorobenzoyl peroxide, and it is used usually in an amount of about 1 to 20 mol., preferably about 2 to 5 mol. relative to the compound (III) or a salt thereof. This reaction can be conducted by using the compound (IV) itself as the solvent or in the presence of a relevant solvent. The solvent is exemplified by water, alcohols (e.g. methanol, ethanol), ethers (e.g. dimethyl ether, diethyl ether, tetrahydrofuran, dioxane, monoglyme, diglyme), nitrile (e.g. acetonitrile), acetone, nitromethane, pyridine, dimethyl sulfoxide, dimethylformamide, hexamethylphosphoramide, sulfolane or a suitable mixture of them. As the photo reaction for causing generation of radical from the peroxide, use is made of, for example, irradiation of ultraviolet ray with a highpressure or low pressure mercury arc lamp. Temperatures of the thermal reaction ranges from about 20°C to the boiling point of the solvent then used (up to about 150°C), preferably from about 50 to 100°C. The reaction time ranges, in any reaction, usually from about 0.5 to 100 hours, preferably from about 1 to 20 hours.

In the case where Z of the compound (IV) is $CH_2OH$, CHO or COOH, the reaction can be carried out in the presence of metal ion using sulfuric acid, water and a peroxide. The amount of the compound (IV) or a salt thereof ranges, generally, from about 1 to 100 mol. relative to the compound (III) or a salt thereof, preferably from about 2 to 40 mol. The amount of metal ion to be used ranges, in general, from about 1 to 25 mol. relative to the compound (III) or a salt thereof, preferably from about 1 to 5 mol.. The metal ion is exemplified by ferrous salt such as ferrous sulfate or ferrous chloride. Examples of the peroxide include a hydrogen peroxide or a tertiary butyl hydroperoxide, which are used usually in an amount of about 1 to 20 mol., preferably about 2 to 5 mol. relative to the compound (III) or a salt thereof. This reaction is carried out preferably in the presence of a suitable solvent. The solvent is exemplified by water, alcohol (e.g. methanol, ethanol), ether (e.g. dimethyl ether, diethyl ether, tetrahydrofuran, dioxane, monoglyme, diglyme), nitrile (e.g. acetonitrile), acetone, nitromethane, pyridine, dimethyl sulfoxide, dimethylformamide, hexamethyl phosphoramide, sulfolane or a suitable

mixture of them. The reaction temperature ranges, usually, from about -10°C to about the boiling point of the solvent then used (up to about 100°C), preferably from about 0 to 50°C. The reaction time ranges usually from about 0.5 to 100 hours, preferably from about 1 to 20 hours.

The compound (I) and (II) or salts thereof of this invention can be produced also by introducing hydroxyl group into the compound (V) or a salt thereof. The reaction of introducing hydroxyl group into the compound (V) or a salt thereof can be carried out by, usually, reacting the compound (V) with oxygen (air) or a peroxide in the presence of metal ion and a base. Sufficient amount of metal ion ranges, generally, from about 0.2 to 50 mol., preferably from about 1 to 10 mol. relative to the compound (V) or a salt thereof. Examples of the metal ion include cupric salts such as cupric chloride or cupric nitrate.

Sufficient amount of the base ranges, generally, from about 1 to 20 mol., preferably from about 1 to 5 mol. relative to the compound (V). Examples of the base include metal bases (e.g. sodium methylate, sodium ethylate, sodium hydroxide, potassium hydroxide, barium hydroxide, lithium hydroxide, sodium carbonate, potassium carbonate, barium carbonate, calcium carbonate, sodium hydrogencarbonate), primary amine (e.g. metylamine, ethylamine, propylamine), secondary amine (e.g. dimethylamine, diethylamine, dipropylamine), tertiary amine (e.g. trimethylamine, triethylamine, triethanolamine), pyridine, $\alpha$-, $\beta$- or $\gamma$-picoline, 2,6-lutidine, 4-dimethylaminopyridine, 4-(1-pyrrolidinyl)pyridine, N,N-dimethylaniline, N,N-diethylaniline, etc.

As the peroxide, mention is made of, for example, hydrogen peroxide and tertiary butyl hydroperoxide, and they are used in an amount of, usually, about 1 to 20 mol., preferably about 2 to 5 mol. relative to the compound (III) or a salt thereof. This reaction is conducted, preferably, in the presence of a suitable solvent. Examples of the solvent include water, alcohol (e.g. methanol, ethanol), ether (e.g. dimethyl ether, diethyl ether, tetrahydrofuran, dioxane, monoglyme, diglyme), nitrile (e.g. acetonitrile), acetone, nitromethane, pyridine, dimethyl sulfoxide, dimethylformamide, hexamethyl phosphoramide, sulfolane or a suitable mixture of them. The reaction temperature ranges from about -20°C to the boiling point of the solvent then used (up to about 100°C), preferably from about -10 to 50°C. The reaction time ranges, usually, from about 1 to 100 hours, preferably from about 5 to 48 hours.

In the case of producing, among the compound (I) and (II) or salts thereof, compounds or salts thereof having, as the substituents of R' , $R^1$, $R^2$, $R^3$, $R^4$ or Y, hydroxy, carboxy, amino, mono $C_{1-6}$ alkyl amino (e.g.methylamino, ethylamino, propylamino, isopropylamino, butylamino), piperidino, sulfo, sulfino, phosphono or dihydroxyboryl, the compound (III) or (IV) or their salts are subjected to reaction, after protecting these groups with <u>per se</u> known protective groups, then the reaction products are subjected to <u>per se</u> known deprotecting reaction to thereby produce the object compound (I) and (II) or salts thereof.

The starting compounds (III), (IV) and (V) or their salts can be prepared in the same manner as described in literature references [W. D. Kingsbury et al., J. Med. Chem., <u>34</u>, 98(1991); JP-02138186; JP-03232888; EP-418099-A].

Respective intermediates of the compounds of this invention and the compounds (I) and (II) of this invention produced by the above-mentioned methods can be isolated from the reaction mixture by a conventional separating means, for example, concentration, solvent extraction, chromatography and recrystallization.

The starting materials (III), (IV) and (V) in the method of this invention and the object compounds (I) and (II) may form salts. As basic salts, mention is made of, for example, alkali metal, alkaline earth metal, non-toxic metal, ammonium or substituted ammonium. Specific examples of them include salts with sodium, potassium, lithium, calcium, magnesium, aluminum, zinc, ammonium, trimethyl ammonium, triethyl ammonium, triethanol ammonium and pyridinium. As acid salts, mention is made of, for example, salts with a mineral acid such as hydrochloric acid, sulfuric acid, nitric acid, phosphoric acid or boric acid, and salts with an organic acid such as oxalic acid, tartaric acid, acetic acid, trifluoroacetic acid, methanesulfonic acid, benzenesulfonic acid, p-toluenesulfonic acid or camphorsulfonic acid.

The compounds (I) and (II) or salts thereof show an inhibitory action against enzymes taking part in topoisomerism in the synthesis of nucleic acid and have an activity of controlling the proliferation of tumor cells. Therefore, these compounds can be used, for the therapy of various tumors, singly or in combination with any other antitumor agent. For example, the compounds (I) and (II) of this invention or pharmaceutically acceptable salts thereof show excellent antitumor effects against mouse tumor cell strains (e.g. P388, L1210, L5178Y, B16 melanoma, MethA, Lewis Lung Carcinoma, S180 sarcoma, Erlich Carcinoma, Colon 26 and 38) and human tumor cell strains (e.g. A549, HL60, KB), and also have an action of decreasing tumors which warm-blooded possess (e.g. leukemia, melanoma, sarcoma, mastocytoma, carcinoma and neoplasia). Therefore, these compounds can be used as antitumor agents for the therapy of tumors of warm-blooded animals possessing tumors, especially mammals (e.g. man, mouse, rat, cat, dog and rabbit).

In the case of using as antitumor agents, the compounds (I) and (II) or salts thereof can be administered orally or non-orally <u>per se</u> or in the form of powder, granules, tablets, capsules, suppositories or injections, prepared by incorporation with pharmaceutically acceptable carriers, excipients and diluents by conventional means. Dosage values will vary with subject animal, disease, symptom, kind of compound or administration route. For example, in the case of oral administration to the above-mentioned warm-blooded animals, the dosage ranges from about 1.0 to 500 mg/kg, preferably from about 10.0 to 200 mg/kg (body weight) per day in terms of the compound of this invention, while in the case of non-oral administration, it ranges from about 1.0 to 200 mg, preferably from 5.0 to 100 mg/kg. Administration as injections includes intramuscular, intraperitoneal, subcutaneous and intravenous injections.

The above-mentioned pharmaceutical compositions can be prepared by per se known methods. In the case of preparing the above-mentioned orally administrable compositions, for example, tablets, there may be incorporated adequately as part of the composition a binder (e.g. hydroxypropyl cellulose, hydroxypropyl methyl cellulose or macrogol), a disintegrator (e.g. starch or carboxymethyl cellulose calcium) and a lubricant (e.g. magnesium stearate or talc), etc.. And, in the case of preparing non-orally administrable compositions, for example, injections, there may be adequately incorporated as part of the composition an isotonizer (e.g. glucose, D-sorbitol, D-mannitol or sodium chloride), a preservative (e.g. benzyl alcohol, chlorobutanol, methyl paraoxybenzoate or propyl paraoxy benzoate), etc.

A specific example of preparing tablets is as follows. About 1.0 to 50 mg of the compound of this invention, 100 to 500 mg of lactose, about 50 to 100 mg of corn starch and about 5 to 20 mg of hydroxypropyl cellulose per tablet were mixed by a conventional manner, and mixture was granulated. The granules were mixed with corn starch and magnesium stearate, and the mixture was subjected to tabletting to give tablets, each weighing about 100 to 500 mg and having about 3 to 10 mm diameter. And, by coating this tablet with an acetone-ethanol mixture dissolving hydroxypropylmethyl cellulose phthalate (about 10 to 20 mg) and castor oil (about 0.5 to 2.0 mg) so that the concentration be about 5 to 10%, the tablet can be made into an enteric coating tablet.

A specific example of preparing injections is as follows. About 2.0 to 50 mg of sodium salt of the compounds (I) and (II) of this invention (1) dissolved in about 2 ml of a physiological saline solution is filled in an ampoule, and the ampoule is sealed, which is sterilized by heating at about 110°C for about 30 minutes, or (2) dissolved in about 2 ml of sterilized distilled water dissolving about 10 to 40 mg mannitol or sorbitol. The solution was lyophilized and the ampoule is sealed to give the desired injection. When the lyophilized injection is used, the ampoule is opened, into which is poured, for example, a physiological saline solution so that the concentration of the compound be about 1.0 to 50 mg/ml, then the injection can be administered subcutaneously, intravenously or intramuscularly.

Elution in a column chromatography in the following Reference Examples and Examples was conducted while monitoring with TLC (Thin Layer Chromatography). In the TLC monitoring, the TLC plate used was $60F_{254}$ manufactured by Merck Co., the developing solvent was the same as the one used for eluting in the column chromatography, and the detection was conducted with a UV detector. The silica gel for the column was silica gel 60 manufactured by Merck Co., (70-230 mesh).

Further, room temperature means 15-25°C.

Abbreviations used in the following Reference Examples and Examples have the following meanings.

J: coupling constant  Hz: hertz
s: singlet  d: doublet
t: triplet  q: quartet
m: multiplet
NMR: Nuclear Magnetic Resonance
IR: Infrared Ray
DMSO: dimethyl sulfoxide

$CDCl_3$: deuteriochloroform
$D_2O$: heavry water
$CD_3OD$: deuteriomethanol
δ: weight %

REFERENCE EXAMPLE 1

7-Ethyl-10H-1,3-dioxolo[4,5-g]pyrano[3',4':6,7]indolizino[1,2-b]quinoline-8,11[7H,13H]dione

In 12 ml of acetic acid was dissolved 300 mg of 4-ethyl-7,8-dihydro-1H-pyrano[3,4-f]indolizine-3,6,10(4H)-trione (J. Med. Chem., vol.23, 554-554(1980)). To the solution was added 200 mg of 2-amino-4,5-methylenedioxybenzaldehyde (J. Chem. Soc., 1736(1951)), and the mixture was stirred for 20 hours at 85°C. The reaction mixture was concentrated, and the concentrate was subjected to a silica gel column chromatography, eluting with a mixture of chloroform-methanol (100:1), to afford 170 mg of the title compound.

$^1$H NMR($CDCl_3$)

1.09(3H,t,J=7.4Hz), 2.10 (2H,m), 3.61(1H,t,J=6.4Hz), 5.25(2H,s), 5.39(1H,d,J=15.2Hz), 5.58(1H,d, J=15.2Hz), 6.19(2H,s), 7.08(1H,s), 7.19(1H,s), 7.33(1H,s), 7.48(1H,s).

IR$\nu_{max}^{KBr}cm^{-1}$ : 1740, 1655, 1600.

REFERENCE EXAMPLE 2

7-Ethyl-7-hydroxy-10H-1,3-dioxolo[4.5-g]pyrano[3',4':6,7]indolizino[1,2-b]quinoline-8,11[7H,13H]-dione

In 75 ml of dimethylformamide was dissolved 240 mg of the compound obtained in the Reference Example 1. To the solution were added 414 mg of copper (II) chloride and 0.81 ml of 25% dimethylamine. The mixture was stirred for 24 hours at room temperature under oxygen atmosphere. The reaction mixture was concentrated, and the concentrate was subjected to a silica gel chromatography, eluting with a mixture of chloroform-methanol (100:2), to afford 54 mg of the title compound.

$^1$H NMR($CDCl_3$)

1.04(3H,t,J=7.3Hz), 1.92(2H,q,J=7.3Hz), 5.25(2H,s), 5.31(1H,d,J=16.5Hz), 5.76(1H,d,J=16.5Hz), 6.21(2H,s), 7.18(1H,s), 7.51(1H,s), 7.64(1H,s), 8.21(1H,s).

IR$\nu_{max}^{KBr}cm^{-1}$ : 3400, 1748, 1660, 1600

REFERENCE EXAMPLE 3

4(S)-4-Ethyl-4-hydroxy-11-trifluoroacetylaminomethyl-1H-pyrano[3',4':6,7]indolizino[1,2-b]quinoline-3,14[4H,12H]-dione

In substantially the same manner as the following Example 1, the title compound (63 mg) was obtained from camptothecin (100 mg) and 2-(trifluoroacetylamino)acetaldehyde (403 mg).

$^1$H NMR(DMSO-$d_6$)

0.90(3H,t,J=7.0Hz), 1.89(2H,q,J=7.0Hz), 5.01(2H,d,J=4.0Hz), 5.44(2H,s), 5.46(2H,s), 6.51(1H,s), 7.37(1H,s), 7.68-7.95(2H,m), 8.20(1H,d,J=8.0Hz), 8.36(1H,d,J=8.2Hz), 10.10-10.30(1H,m).

IR$\nu_{max}^{KBr}cm^{-1}$ :3430, 1720, 1655, 1600.

REFERENCE EXAMPLE 4

11-Benzyloxycarbonylaminomethyl-4(S)-4-ethyl-4-hydroxy-1H-pyrano[3',4':6,7]indolizino[1,2-b]-quinoline-3,14[4H,12H]-dione

In substantially the same manner as the following Example 1, the title compound (73 mg) was obtained from camptothecin (77 mg) and 2-(benzyloxycarbonylamino)acetaldehyde (384 mg).

$^1$H NMR($CDCl_3$)

1.04(3H,t,J=7.6Hz), 1.63(2H,q,J=7.6Hz), 4.92(2H,d,J=6.0Hz), 5.11(2H,s), 5.30(1H,d,J=16.6Hz), 5.44(2H,s), 5.70(1H,d,J=16.6Hz), 6.94(1H,t,J=6.0Hz), 7.34(1H,s), 7.61-7.88(2H,m), 8.13-8.31(2H,m).

IR$\nu_{max}^{KBr}cm^{-1}$ : 3400, 1745, 1720, 1655, 1595.

REFERENCE EXAMPLE 5

11-Aminomethyl-4(S)-4-ethyl-4-hydroxy-1H-pyrano[3',4':6,7] indolizino[1,2-b]quinoline-3,14[4H,12H]-dione

To a solution of the compound of the Reference Example 3 (70 mg) in acetic acid (1.5 ml) was added a 47% aqueous solution of hydrogen bromide (0.3 ml), and the mixture was stirred for 3 hours at 100°C. The reaction mixture was left standing for cooling, to which was then added a saturated aqueous solution of sodium hydrogencarbonate. The mixture was subjected to extraction with chloroform. The extract solution was washed with a saturated aqueous saline solution, then dried over anhydrous sodium sulfate, from which was distilled off the solvent to afford the title compound (26 mg).

Or, also from the compound of Reference Example 4 (22 mg), the title compound (16 mg) was obtained in substantially the same manner as described above.
$^1$H NMR (DMSO-$d_6$)
0.91(3H,t,J=7.0Hz), 1.89(2H,q,J=7.0Hz), 4.46(2H,s), 5.38-5.56(4H,m), 6.43-6.52(1H,m), 7.37(1H,s), 7.64-8.38(4H,m), 8.20(1H,d,J=8.0Hz), 8.36(1H,d,J=8.2Hz), 10.10-10.30(1H,m).
$IR\nu_{max}^{KBr}cm^{-1}$ : 3400, 1750, 1655, 1600

REFERENCE EXAMPLE 6

11-Aminomethyl-4(S)-4-ethyl-4-hydroxy-1H-pyrano[3',4':6,7] indolizino[1,2-b]quinoline-3,14[4H,12H]-dione hydrochloride

To the compound of the Reference Example 5 (5 mg) was added a 20% hydrochloric acid - ethanol solution (1 ml). The mixture was stirred for 5 hours at room temperature. The reaction mixture was then concentrated to afford the title compound (5 mg).
$^1$H NMR (DMSO-$d_6$)
0.90(3H,t,J=7.4Hz), 1.89(2H,q,J=7.4Hz), 4.58-4.86(2H,m), 5.45(2H,s), 5.65(2H,s), 7.38(1H,s), 7.75-8.00(2H,m), 8.25(1H,d,J=8.2Hz), 8.45(1H,d,J=8.4Hz), 8.62-8.82(3H,m).
$IR\nu_{max}^{KBr}cm^{-1}$ : 3430, 1740, 1650, 1600.

EXAMPLE 1

14-N-t-Butoxycarbonylaminomethyl-7-ethyl-7-hydroxy-10H-1,3-dioxolo[4,5-g]pyrano[3',4':6,7]indolizino[1,2-b] quinoline -8,11[7H,13H]-dione

In 2 ml of water was dissolved 80 mg of $FeSO_4.7H_2O$, to which was added 100 mg of 2-(N-t-butoxycarbonyl)aminoaldehyde. In the solution were suspended 20 mg of the compound obtained in the Reference Example 2 and 1.3 ml of acetic acid. To the suspension was further added 0.27 ml of conc. sulfuric acid to make a solution. To the solution was added dropwise slowly 15 mg of 30% $H_2O_2$ under ice-cooling. The mixture was then stirred for one hour at room temperature. The reaction mixture was poured into 10 ml of ice-water, which was subjected to extraction with chloroform. From the extract was distilled off the solvent under reduced pressure. The residue was subjected to a silica gel column chromatography, eluting with chloroform, to afford 8 mg of the title compound.
$^1$H NMR($CDCl_3$+$CD_3OD$)
1.03(3H,t,J=7.4Hz), 1.48(9H,s), 1.91(2H,q,J=7.4Hz), 4.75(2H,d,J=7.1Hz), 5.27(1H,d,J=17.4Hz), 5.29 (2H,s), 5.68(1H,d,J=17.4Hz), 6.21(2H,s), 7.42(1H,s), 7.46(1H,s), 7.56(1H,s)
$IR\nu_{max}^{KBr}cm^{-1}$ : 3400, 1750-1730, 1660, 1600.

EXAMPLE 2

14-Aminomethyl-7-ethyl-7-hydroxy-10H-1,3-dioxolo[4,5-g] pyrano[3',4':6,7]indolizino[1,2-b]quinoline-8,11[7H, 13H]-dione·trifluoroacetic acid salt

In 1 ml of dichloromethane was suspended 8 mg of the compound obtained in the Example 1. To the suspension was added dropwise 0.1 ml of trifluoroaceticacid, and the mixture was stirred for 3 hours at room temperature. The reaction mixture was concentrated under reduced pressure. The concentrate was dissolved in water, which was lyophilized to afford 6 mg of the title compound.
$^1$H NMR($D_2O$)
0.71(3H,t,J=7.3Hz), 1.72(2H,q,J=7.3Hz), 4.50-5.45(6H,m), 6.21(2H,s), 6.69(1H,s), 7.02(1H,s), 7.13(1H,s)

EXAMPLE 3

14-(N-t-Butoxycarbonylamino)ethyl-7-ethyl-7-hydroxy-10H-1,3-dioxolo[4,5-g]pyrano[3',4':6,7]indolizino[1,2-b] quinoline-8,11[7H,13H]-dione

In substantially the same manner as the Example 1, the title compound (9 mg) was prepared from the compound (20 mg) of the Reference Example 2 and 3-(N-t-butoxycarbonylamino)propionaldehyde (150 mg).
$^1$H NMR($CDCl_3$+$CD_3OD$)
1.03(3H,t,J=7.4Hz), 1.48(9H,s), 1.91(2H,q,J=7.4Hz), 3.30-4.11(4H,m), 5.21(2H,s), 5.28(1H,d,J=17.4Hz), 5.74(1H,d,J=17.4Hz), 6-19(2H,s), 7.42-7.66(3H,m).

EXAMPLE 4

14-Aminoethyl-7-ethyl-7-hydroxy-10H-1,3-dioxolo[4,5-g] pyrano[3',4':6,7]indolizino[1,2-b]quinoline-8,11[7H, 13H]-dione·trifluoroacetic acid salt

In substantially the same manner as the Example 2, the title compound (4 mg) was prepared from the compound (6 mg) of the Example 3.

$^1H$ NMR($D_2O$)

1.03(3H,t,J=7.4Hz), 1.97(2H,q,J=7.4Hz), 3.30-4.02(4H,m), 5.31-6.01(4H,m), 6.41(2H,s), 7.58(1H,s), 7.63(1H,s), 7.98(1H,s).

EXAMPLE 5

7-Ethyl-7-hydroxy-14-(N-trifluoroacetylamino)methyl-10H-1,3-dioxolo[4,5-g]pyrano[3',4':6,7]indolizino[1,2-b] quinoline-8,11[7H,13H]-dione

In substantially the same manner as the Example 1, the title compound (14 mg) was prepared from the compound (40 mg) of the Reference Example 2 and 2-(N-trifluoroacetylamino)acetaldehyde (600 mg).

$^1H$ NMR ($CD_3OD+CDCl_3$)

1.03(3H,t,J=7.4Hz), 1.97(2H,q,J=7.4Hz), 4.81-5.70(6H,m), 6.22(2H,s), 7.38-7.67(3H,m).

EXAMPLE 6

7-Ethyl-7-hydroxy-14-hydroxymethyl-10H-1,3-dioxolo[4,5-g]pyrano[3',4':6,7]indolizino[1,2-b]quinoline-8.11 [7H,13H]-dione

In 10 ml of methanol was dissolved the compound (20 mg) of the Reference Example 2, to which were added 0.15 ml of conc. sulfuric acid and 30 mg of benzoyl peroxide. The mixture was stirred for 9 hours at 70°C. The reaction mixture was poured into 10 ml of ice-water, which was subjected to extraction with chloroform. From the extract, the solvent was distilled off under reduced pressure. The residue was subjected to a silica gel column chromatography, eluting with chloroform-methanol (10:1), to afford the title compound (7 mg).

$^1H$ NMR ($CD_3OD+CDCl_3$)

1.01(3H,t,J=7.4Hz), 1.97(2H,q,J=7.4Hz), 5.21-5.80(6H,m), 6.20(2H,s), 7.18-7.67(3H,m).

EXAMPLE 7

14-(N-Benzyloxycarbonylamino)methyl-7-ethyl-7-hydroxy-10H-1,3-dioxolo[4,5-g]pyrano[3',4':6,7]indolizino[ 1,2-b]quinoline-8,11[7H,13H]-dione

In substantially the same manner as the Example 1, the title compound (54 mg) was obtained from the compound of the Reference Example 2 (150 mg) and 2-(N-benzyloxycarbonylamino)acetaldehyde (1.43 g).

$^1H$ NMR($CD_3OD+CDCl_3$)

1.03(3H,t,J=7.4Hz), 1.91(2H,q,J=7.4Hz), 4.72-4.83(2H,m), 5.11(2H,s), 5.28(1H,d,J=16.2Hz), 5.33(2H,s), 5.66(2H,d,J=16.2Hz), 6.21(2H,s), 6/91-7.08(1H,m), 7.34(5H,s), 7.45(1H,s), 7.52(1H,s), 7.58(1H,s).

$$IR\nu_{max}^{KBr}\ cm^{-1}: 3400,\ 1710,\ 1650.$$

EXAMPLE 8

14-(N-Benzyloxycarbonylamino)ethyl-7-ethyl-7-hydroxy-10H-1,3-dioxolo[4,5-g]pyrano[3',4':6,7]indolizino[1, 2-b]quinoline-8,11[7H,13H]-dione

In substantially the same manner as the Example 1, the title compound (26 mg) was obtained from the compound of the Reference Example 2 (43 mg) and 2-(N-benzyloxycarbonylamino)propionaldehyde (125 mg).

$^1H$ NMR(DMSO-$d_6$)

0.88(3H,t,J=7Hz), 1.87(2H,q,J=7Hz), 3.10-4.21(4H,m), 4.96(2H,s), 5.23(2H,s), 5.43(2H,s), 6.28(2H,s), 6.50(1H,s), 7.17-7.88(8H,m).

EXAMPLE 9

14-Aminoethyl-7-ethyl-7-hydroxy-10H-1,3-dioxolo[4,5-g] pyrano[3',4':6,7]indolizino[1,2-b]quinoline-8,11[7H,13H]-dione·hydrochloride

The compound of the Example 3 (1.0 g) was suspended in 180 ml of methanol, to which was added, under ice-cooling, 30 ml of 4N hydrogen chloride - ethyl acetate solution. The mixture was stirred at room temperature overnight. The solvent was distilled off to leave crude crystals, followed by recrystallization from methanol-diethyl ether to afford 0.8 g of the title compound as a yellow powdery product.

$^1H$ NMR($CD_3OD$)

0.99(3H,t,J=7.3Hz), 1.95(2H,q,J=7.3Hz), 3.30-3,33(2H,m), 3.43-3.48(2H,m), 5.26(2H,s), 5.37(1H,d,J=16.0Hz), 5.58(1H,d,J=16.0Hz), 6.24(1H,s), 6.26(1H,s), 7.44(1H,s), 7.52(1H,s), 7.53(1H,s).

$$IR\nu^{KBr}_{max}: cm^{-1}\ 3450,\ 1730,\ 1655$$

FORMULATION EXAMPLE 1

The compound of the Example 9 was dissolved in distilled water. The solution was subjected to filtration. The filtrate was lyophilized to give a powdery product. 25 mg each of the product was put in vials under aseptic conditions. The vials were sealed and stored under the shade of light.

FORMULATION EXAMPLE 2

| | |
|---|---|
| Compound of the Example 2 | 25 mg |
| Lactose | 98 mg |
| Hydroxypropyl cellulose | 3 mg |
| Crystalline cellulose | 20 mg |
| Talc | 2 mg |
| Magnesium stearate | 2 mg |

The above ingredients were blended and the mixture was subjected to direct compression using a tablet machine to provide tablets, each weighing 150 mg.

The invention may be embodied in other specific forms without departing from the spirit or essential characteristics thereof. The present embodiment is therefore to be considered in all respects as illustrative and not restrictive, the scope of the invention being indicated by the appended claims rather than by the foregoing description and all changes which come within the meaning and range of equivalency of the claims are therefore intended to be embraced therein.

## Claims

1. A compound of the general formula:

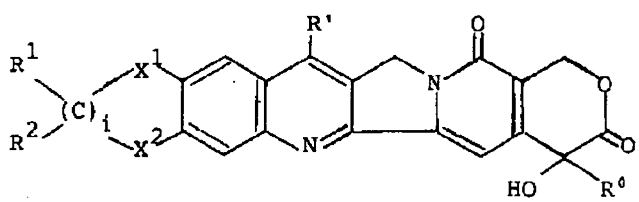

(I)

wherein R' stands for an aliphatic hydrocarbon group substituted with a hydrophilic group; $R^0$ stands for a lower alkyl group; $R^1$ and $R^2$ independently stand for a hydrogen atom or an optionally substituted lower hydrocarbon group; $X^1$ and $X^2$ independently stand for - O-, -S(O)$_\kappa$- ($\kappa$ denotes a whole number of 0 to 2),

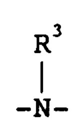

($R^3$ stands for a hydrogen atom or an optionally substituted lower hydrocarbon group) or an optionally substituted methylene group; i denotes a whole number of 1 to 3; and $R^1$ and $R^2$ may be different from each other at the repetition of the number i, or a salt thereof.

2. A compound as claimed in claim 1, which is a compound of the general formula:

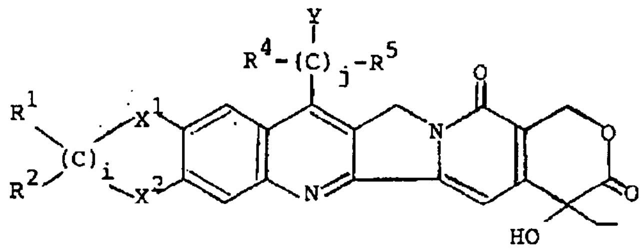

(II)

wherein $R^1$, $R^2$, $R^4$ and $R^5$ independently stand for a hydrogen atom or an optionally substituted lower hydrocarbon group; $X^1$ and $X^2$ independently stand for - O-, $-S(O)_\kappa-$ ($\kappa$ denotes a whole number of 0 to 2),

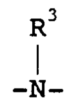

($R^3$ stands for a hydrogen atom or an optionally substituted lower hydrocarbon group) or an optionally substituted methylene group; Y stands for $-O-R^6$ ($R^6$ stands for a hydrogen atom or an organic group having a water-soluble substituent) or

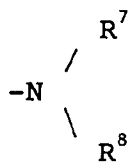

($R^7$ and $R^8$ independently stand for a hydrogen atom, an optionally substituted lower hydrocarbon group or an organic group having a water-soluble substituent, or they may form a heterocyclic group taken together with the adjacent nitrogen atom); i and j respectively denote a whole number of 1 to 3; $R^1$, $R^2$, $R^4$ and $R^5$ may be different from one another at the repetition of the number i or j, or a salt thereof.

3. A compound as claimed in claim 1, wherein the hydrophilic group stands for -OQ, -CN, $-NQ_2$, $-\overset{\oplus}{N}Q_3$, an cyclic amino group, -OCOQ, -OCONHQ, -COOQ, -NHCOQ, $-NHSO_2Q$, $-NHPO_2Q$, -NHCONHQ, -NHCOOQ, $-SO_3Q$ or $-OSO_3H$ wherein Q stands for a hydrogen atom or an optionally substituted lower hydrocarbon group.

4. A compound as claimed in claim 1, wherein the aliphatic hydrocarbon group is a straight-chain or branched $C_{1-6}$ alkyl group.

5. A compound as claimed in claim 1, wherein the lower alkyl group is a $C_{1-6}$ alkyl group.

6. A compound as claimed in claim 1 or 2, wherein the optionally substituted lower hydrocarbon group is a $C_{1-6}$ alkyl, $C_{2-6}$ alkenyl, $C_{2-6}$ alkynyl, $C_{3-6}$ cycloalkyl, phenyl or benzyl which may be substituted with one to three substituents selected from the group consisting of a halogen, nitro, cyano, hydroxy, carboxy, carbamoyl, N-mono-$C_{1-6}$ alkylcarbamoyl, N,N-di-$C_{1-6}$ alkylcarbamoyl, $C_{1-6}$ alkoxy, phenoxy, benzyloxy, amino, mono-$C_{1-6}$ alkylamino, di-$C_{1-6}$ alkylamino, trifluoromethyl, $C_{1-5}$ alkanoyl, $C_{1-3}$ alkoxy-$C_{1-3}$ alkyl, $C_{1-6}$ alkylthio, sulfamoyl, N-mono-$C_{1-6}$ alkylsulfamoyl, N,N-di-$C_{1-6}$ alkylsulfamoyl, $C_{1-6}$ alkylsulfinyl and $C_{1-6}$ al-

kylsulfonyl.

**7.** A compound as claimed in claim 1 or 2, wherein the optionally substituted methylene group is a methylene group which may be substituted with one to three substituents selected from the group consisting of a halogen, nitro, cyano, hydroxy, carboxy, carbamoyl, N-mono-$C_{1-6}$ alkylcarbamoyl, N,N-di-$C_{1-6}$ alkylcarbamoyl, $C_{1-6}$ alkoxy, phenoxy, benzyloxy, amino, mono-$C_{1-6}$ alkylamino, di-$C_{1-6}$ alkylamino, trifluoromethyl, $C_{1-5}$ alkanoyl, $C_{1-3}$ alkoxy-$C_{1-3}$ alkyl, $C_{1-6}$ alkylthio, sulfamoyl, N-mono-$C_{1-6}$ alkylsulfamoyl, N,N-di-$C_{1-6}$ alkylsulfamoyl, $C_{1-6}$ alkylsulfinyl and $C_{1-6}$ alkylsulfonyl.

**8.** A compound as claimed in claim 1, wherein the hydrophilic group is $-OQ^1$, $-OCOQ^1$ or $-OCONHQ^1$ wherein $Q^1$ is (i) a hydrogen atom, or (ii)a $C_{1-6}$ alkyl, $C_{2-6}$ alkenyl, $C_{2-6}$ alkynyl, $C_{3-6}$ cycloalkyl or benzyl group which has one to three substituents selected from the group consisting of a hydroxy, carboxy, amino, mono-$C_{1-6}$ alkylamino, di-$C_{1-6}$ alkylamino, 5 to 6-membered cyclic amino, $C_{1-3}$ alkoxy-$C_{1-3}$ alkyl, sulfo, sulfino, phosphono and dihydroxyboryl.

**9.** A compound as claimed in claim 2, wherein Y is $-OR^6$ wherein $R^6$ is (i)a hydrogen atom or (ii) a $C_{1-4}$ alkyl or benzyl group which has one to three substituents selected from the group consisting of a hydroxy, carboxy, amino, mono-$C_{1-6}$ alkylamino, di-$C_{1-6}$ alkylamino, 5- to 6-membered cyclic amino, $C_{1-3}$ alkoxy-$C_{1-3}$ alkyl, sulfo, sulfino, phosphono and dihydroxyboryl.

**10.** A compound as claimed in claim 2, wherein Y is $-NR^{7'}R^{8'}$, $-NHCOR^{7'}$, $-NHSO_2R^{7'}$, $-NHPO_2R^{7'}$, $-NHCONHR^{7'}$, $- NHCOOR^{7'}$ ($R^{7'}$ and $R^{8'}$ independently stand for (i) a hydrogen atom or (ii) a $C_{1-6}$ alkyl, $C_{2-6}$ alkenyl, $C_{2-6}$ alkynyl, $C_{3-6}$ cycloalkyl or benzyl group which has one to three substituents selected from the group consisting of hydroxy, carboxy, amino, mono-$C_{1-6}$ alkylamino, di-$C_{1-6}$ alkylamino, 5- to 6- membered cyclic amino, $C_{1-3}$ alkoxy-$C_{1-3}$ alkyl, sulfo, sulfino, phosphono and dihydroxyboryl).

**11.** A compound as claimed in claim 2, wherein Y is an amino, $-NHCOR^{7''}$ or $-NHCOOR^{7''}$ wherein $R^{7''}$ stands for (i) a hydrogen atom or (ii) a $C_{1-4}$ alkyl or benzyl group which has one to three substituents selected from the group consisting of a hydroxy, carboxy, amino, mono-$C_{1-6}$ alkylamino, di-$C_{1-6}$ alkylamino, 5- to 6- membered cyclic amino, $C_{1-3}$ alkoxy-$C_{1-3}$ alkyl, sulfo, sulfino, phosphono, phenyl and dihydroxyboryl.

**12.** A compound as claimed in claim 2, wherein $R^1$ and $R^2$ independently stand for a hydrogen atom or a $C_{1-3}$ alkyl group; $X^1$ and $X^2$ independently stand for -O-, -S- or -NH-; i denotes 1 or 2; and j denotes 1 to 3.

**13.** A compound as claimed in claim 2, wherein both $R^1$ and $R^2$ stand for a hydrogen atom; $X^1$ and $X^2$ stand for - O-; j denotes 1 or 2; and i denotes 1.

**14.** A compound as claimed in claim 13, wherein $R^4$ and $R^5$ independently stand for a $C_{1-6}$ alkyl, $C_{2-6}$ alkenyl, $C_{3-6}$ cycloalkyl or phenyl which may be substituted with one to three substituents selected from the group consisting of a halogen, nitro, cyano, hydroxy, carboxy, carbamoyl, N-mono $C_{1-6}$ alkylcarbamoyl, N,N-di-$C_{1-6}$ alkylcarbamoyl, $C_{1-6}$ alkoxy, phenoxy, benzyloxy, amino, mono $C_{1-6}$ alkylamino, di-$C_{1-6}$ alkylamino, trifluoromethyl, $C_{1-5}$ alkanoyl, $C_{1-3}$ alkoxy-$C_{1-3}$ alkyl, $C_{1-6}$ alkylthio, sulfamoyl, N-mono $C_{1-6}$ alkylsulfamoyl, N,N-di-$C_{1-6}$ alkylsulfamoyl, $C_{1-6}$ alkylsulfinyl and $C_{1-6}$ alkylsulfamoyl; Y stands for (1) $-OR^6$ wherein $R^6$ stands for (i) a hydrogen atom or (ii) a $C_{1-4}$ alkyl or benzyl group which has one to three substituents selected from the group consisting of a hydroxy, carboxy, amino, mono-$C_{1-6}$ alkylamino, di-$C_{1-6}$ alkylamino, 5- to 6-membered cyclic amino, $C_{1-3}$ alkoxy-$C_{1-3}$ alkyl, sulfo, sulfino, phosphono and dihydroxyboryl, (2) $-NH_2$, $- NHCOR^{7'}$ or $-NHCOOR^{7'}$ wherein $R^{7'}$ stands for (i) a hydrogen atom or (ii) a $C_{1-4}$ alkyl or benzyl group which has one to three substituents selected from the group consisting of a hydroxy, carboxy, amino, mono-$C_{1-6}$ alkylamino, di-$C_{1-6}$ alkylamino, 5- to 6-membered cyclic amino, $C_{1-3}$ alkoxy-$C_{1-3}$ alkyl, sulfo, sulfino, phosphono and dihydroxyboryl.

**15.** A compound as claimed in claim 13, wherein $R^4$ and $R^5$ independently stand for a hydrogen atom or a $C_{1-3}$ alkyl group.

**16.** A compound as claimed in claim 13, wherein both $R^4$ and $R^5$ stand for a hydrogen atom.

**17.** A compound as claimed in claim 13, wherein Y stands for an amino group or a hydroxyl group.

**18.** A compound as claimed in claim 13, wherein Y stands for an amino group.

**19.** A compound as claimed in claim 2, wherein $R^1$ and $R^2$ independently stand for a hydrogen atom; $X^1$ and $X^2$ independently stand for -O-; $R^4$ and $R^5$ independently stand for a hydrogen atom; Y stands for -OH, $-NH_2$, $-NHCOR^{7'''}$, $-NHCOOR^{7'''}$ (wherein $R^{7'''}$ stands for a hydrogen atom, $C_{1-4}$ alkyl or benzyl group); i is 1; j is 1 or 2.

**20.** A method of producing a compound as claimed in claim 1, which comprises reacting a compound of the formula:

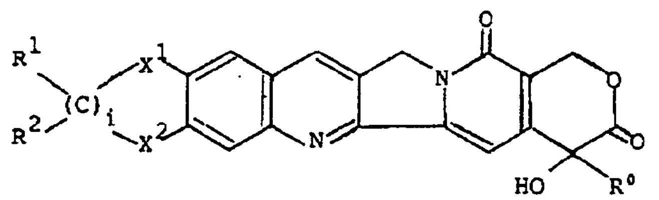

(III)

wherein $R^0$, $R^1$, $R^2$, $X^1$, $X^2$ and i are of the same meaning as defined in claim 1 or a salt thereof with a compound represented by the formula:

R' - Z (IV)

wherein Z stands for a hydrogen atom, $CH_2OH$, CHO or COOH and R' is as defined in claim 1, or a salt or a reactive derivative thereof.

**21.** A method of producing a compound as claimed in claim 1, which comprises introducing a hydroxyl group into a compound of the general formula:

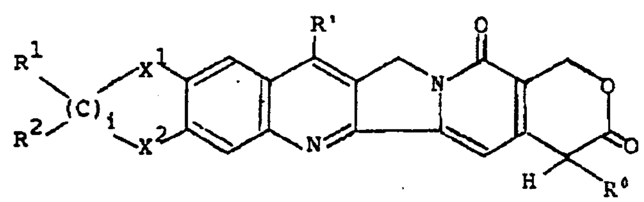

(V)

wherein the symbols are of the same meaning as defined in claim 1 or a salt thereof.

**22.** A compound as claimed in claim 13, which is 14-aminomethyl-7-ethyl-7-hydroxy-10H-1,3-dioxolo[4,5-g] pyrano[3',4':6,7]indolizino[1,2-b]quinoline-8,11[7H,13H]-dione or its trifluoroacetate.

**23.** A compound as claimed in claim 13, which is 14-aminoethyl-7-ethyl-7-hydroxy-10H-1,3-dioxolo[4,5-g]pyano[3',4':6,7]indolizino[1,2-b]quinoline-8,11[7H,13H]-dione or its trifluoroacetate.

**24.** A compound as claimed in claim 13, which is 7-ethyl-7-hydroxy-14-hydroxmethyl-10H-1,3-dioxolo[4,5-g]pyrano[3',4':6,7]indolizino[1,2-b]quinoline-8,11[7H,13H]-dione.

**25.** A compound as claimed in claim 12, which is 14-aminoethyl-7-ethyl-7-hydroxy-10H-1,3-dioxolo[4,5-g]pyrano[3',4':6,7]indolizino[1,2-b]quinoline-8,11[7H,13H]-dione or its hydrochloride.

**26.** A compound as claimed in claim 1 or 2, wherein an absolute configuration of the lactone ring is S-configuration.

**27.** A compound as claimed in claim 1 or 2, which is a racemic mixture.

**28.** An antitumor composition which comprises an effective amount of a compound as claimed in claim 1 and a physiologically acceptable carrier.

29. A composition as claimed in claim 28 which comprises an effective amount of a compound as defined in claim 19.

30. A topoisomerase I inhibitor which contains an effective amount of a compound as claimed in claim 1 and a physiologically acceptable carrier.

31. A method for treating tumor in mammals which comprises administrating to a subject suffering therefrom an effective amount of a compound as claimed in claim 1 with a physiologically acceptable carrier.

32. A method for suppressing topoisomerase I activity in mammals which comprises administrating to a subject in need an effective amount of a compound as claimed in claim 1 with a physiologically acceptable carrier.

33. Use of a compound as claimed in claim 1 as an effective component in the preparation of an antitumor composition.

34. A compound as claimed in claim 2, wherein the heterocyclic group stands for a pyrrolidinyl or morpholino group.

35. A compound as claimed in claim 18, wherein the amino group may be protected by a formyl, acetyl, chloroacetyl, trifluoroacetyl, benzoyl, phthaloyl, 2,2,2-trichloroethyloxycarbonyl, tert-butyloxycarbonyl, benzyloxycarbonyl or 7-fluorenyloxycarbonyl group.

36. A compound as claimed in claim 18, wherein the amino group may be protected by a tert-butylorycarbonyl, benzyloxycarbonyl or trifluoroacetyp group.